# EUROPEAN PATENT SPECIFICATION

(11) **EP 1 272 146 B1**
(45) Date of publication and mention of the grant of the patent: **18.01.2012**
(21) Application number: 01920075.7
(22) Date of filing: 09.04.2001
(51) Int. Cl.: A61K 6/06, C04B 28/06

(54) **CHEMICALLY BOUND CERAMIC PRODUCT AND METHOD FOR ITS PRODUCTION**
CHEMISCH GEBUNDENES KERAMIKPRODUKT UND VERFAHREN ZU DESSEN HERSTELLUNG
PRODUIT CERAMIQUE A LIAISON CHIMIQUE ET PROCEDE DE PRODUCTION

(30) Priority: 11.04.2000 SE 0001322
(43) Date of publication of application: 08.01.2003
(73) Proprietor: Doxa Aktiebolag, 754 51 Uppsala (SE)
(72) Inventor: HERMANSSON, Leif, 26042 Mölle (SE); SAHLBERG, Lena, S-754 49 Uppsala (SE)
(74) Representative: Lindgren, Anders
(86) International application number: PCT/SE2001/000781
(87) International publication number: WO 2001/076535

(56) References cited:
- EP-A2- 0 559 627
- WO-A1-90/11066

## Description

### TECHNICAL FIELD

The present application relates to a raw compact comprising a binding phase of one or more powdered binding agents, which raw compact has the capacity, following saturation with a liquid reacting with the binding agents, to form a chemically bound ceramic material, and which raw compact preferably has a degree of compactness of 55-67 percent by volume solid phase. The primary application of the ceramic material is as dental filling material. The invention also relates to a method for producing said ceramic material.

### BACKGROUND TO THE INVENTION

The present invention relates to binding agent systems of the cement system type, in particular the system CaO-Al₂O₃-(SiO₂)-H₂O. This system is used in the construction industry for exceptionally hard and tough environments, i.e. acid environments with high mechanical stress (R J Mangabhai, Calcium Aluminate Cements, Conference volume, E & F N Spon, London, 1990). By applying rupture mechanical attach methods and advanced powder technology to the system, the generally good profile of features of the base system can be improved considerably. Studies carried out according to the invention and previous works (SE 463 493 and 502 987) have produced a result that indicates great potential for the system for strong and acid-resistant materials such as dental filling materials. No dental filling material existing today meets all the requirements as regards biocompatibility, aesthetics and function that may be set by patients and dental service staff. The situation for various dental filling materials can be summarized as follows: Amalgam has generally good durability, but has shortcomings as far as biocompatibility and aesthetics are concerned Plastic composites have good workability, but shortcomings with regard to erosion and corrosion and handling for staff (allergy problems have arisen). Plastic composites shrink on hardening, which gives rise to the risk of cracks forming and the onset of decay in the long run. Glass ionomers have a good bond with dentine and enamel, but shortcomings with regard to corrosion and strength. Silicate cement has good compressive strength and aesthetics, but suffers from corrosion and strength problems.

EP 1 121 087 generally disclosed a chemically bound ceramic material, the binder phase of which essentially consists of a cement based system, especially the system CaO-Al₂O₃-(SiO₂)-H₂O. Said material additionally comprises one or more expansion compensating additives adapted to give the material dimension stable long time properties. The material is primarily intended for use as a dental filling material.

Various types of inlay have good mechanical properties, but are labour-intensive and require gluing.

Below is a description of the requirements which should generally be set for a new, practical dental filling material; good handling ability with easy applicability in a cavity, moulding which permits good modellability, hardening/solidification which is sufficiently fast for filling work and serviceable directly following the visit to the dentist. Furthermore, high strength and corrosion resistance exceeding that of existing filling materials are required, good biocompatibility, good aesthetics and safe handling for staff without allergy-inducing or toxic additives in the materials. In addition, good long-term characteristics with regard to dimensional stability are wanted. This is a problem in particular if the material expands over time, which can cause disastrous tooth breakages as a result.

In SE 463 493 it has been described how a chemically bound ceramic material, for e.g. dental purposes, can be caused to have enhanced strength characteristics in that a powder body consisting of one or more hydraulic binding agents and possible ballast material is compacted at such a high external pressure and so low a temperature that a closely connected raw compact is obtained without sintering reactions on compaction . In this raw compact, the filling density has increased to at least 1.3 times the initial filling density, which is defined as the filling density which is achieved by shaking, vibration and/or light packing of the loose powder in a container. The user of the material prepares the same by saturating the raw compact with a hydrating liquid prior to application of the material or in situ in a cavity, e.g. a tooth cavity.

The material produced according to SE 463 493 has indeed proved to satisfy most requirements that can be set according to the above for dental filling material. However, it has been found that problems can occur with dimensional changes, especially long-term expansion, which according to the above can have disastrous consequences in connection with dental fillings. There is room for further development of the material and its manufacture with regard to other aspects also. Such aspects are for example the degree of compactness of the raw compact, which influences the durability of the raw compact in various directions, workability on application, and the strength of the finished product. Other aspects concern optimization of the raw compact's dimensions and shape and how it is moistened in connection with application. It has been found that problems can occur when the material is hydrated too quickly. There is not then sufficient time, after the raw compact has been mixed with hydration liquid, to mould the material and/or cut into the same, before it solidifies. This problem is accentuated with any accelerator in the hydration liquid. However, the use of accelerator is desirable in itself, since it means that the product quickly becomes polishable, and since the patient can leave the clinic quickly and eat etc.

One aspect that is related to the use of a raw compact for dental fillings is the question of how the raw compact is to be handled purely physically. An instrument is required for gripping the raw compact and transporting it to the cavity that is intended to be filled. A compaction device is likewise required for compaction of the raw compact provided with liquid in the cavity.

Following SE 463 493, it has been shown according to Swedish patent 502 987, that for cement systems complete hydration (which would then lessen the risk of dimensional changes) can take place if complete soaking and subsequent compacting of the cement system take place using a specially designed stopper. However, the method does not prevent dimensional changes which take place afterwards and which are related to phase transformations of hydrate or reactions with the surrounding atmosphere (for example, exhalation air with a higher carbon dioxide content), or other reactions. These reactions and related dimensional changes become more obvious in cases where a high degree of compaction is used in the production of the material. However, a higher degree of packing is normally sought, as this generally gives greater strength. The method according to SE 502 987 may also require a good deal of training before an individual dentist uses it to perfection, which may cause the individual dentist to hesitate to use the technique.

In Yan et al, Characteristics of shrinkage compensation expansive cement containing prehydrated high alumina cement-based expansive additive, Cement and Concrete Research, Vol 24, p 267-276 (1990), the use of calcium aluminate's tendency to expand is described. This article and related works on expansive cements describe the possibilities of making standard cement expand or shrink less using calcium aluminates amongst other things, but do not touch on the problems of long-term expansion of highly compacted cement systems and control of the expansion of calcium aluminates at very low levels, which is a prerequisite for the use of these binding agent systems in applications according to the present invention.

Other immediate works and patents which do not, however, concern the principal field of the present invention are for example SE-B-381 808, EP-A-0 024 056 and EP-A-0115 058. DE 5 624 489 and US-A-4 689 080.

### DISCLOSURE OF THE INVENTION

One object of the present invention is to provide a raw compact of the type stated in the preamble of claim 1, which raw compact following hydration gives a chemically bound ceramic material that has dimensionally stable long-term properties. The raw compact must also satisfy the requirements stated above for workability and durability, and be easy to handle in connection with its moistening and application in a cavity, e.g. a tooth cavity. The ceramic material formed should also, for dental applications, satisfy the demands that are made on such material according to the above.

This is achieved according to the invention by means of the characterizing features of claim 1.

### COMPOSITION OF THE POWDER MATERlAL INCLUDING ADDITIVES

Apart from good mechanical properties, chemical attributes are important for dental applications. In a significant aspect of the invention, calcium aluminates. i.e. double oxides of CaO (calcium oxide) and Al₂O₃ (aluminium oxide) - here and below termed the CA system, which reacts with water, forming calcium aluminate hydrates - are used as the main binding phase. This hydration reaction constitutes the actual setting and hardening process. Conventionally, some type of aggregate (filler particle) is added to the calcium aluminate cement, principally for economic reasons. According to the invention, the choice of the CA cement system, combined with another cement system or phase which interacts with the aluminate cement, or combined with the addition of porous aggregates or soft materials, produces a dimensional change which is less than approx. 0.20 % linearly, often less than 0.10 %. In special cases, the dimensional change may be close to zero expansion.

According to a first embodiment of the invention, the CA system can be used as the only main binding phase or with the addition of another cement binding phase in amounts of less than 30 percent by volume. Admixtures of ordinary Portland cement (OPC cement) or fine-grained silicon dioxide are used advantageously. Since the calcium aluminate cement has a tendency to expand more strongly on harder packing, combinations ofCA cement and another phase of said type, with a tendency to shrink, can produce reduced dimensional changes. The CA cement should be present in dental applications as the main phase in the binding phase, as the CA phase contributes to high strength and acid resistance.

It has proved to be the case that the theories regarding reasons for dimensional changes which were put forward in connection with Swedish patent 502 987, i.e. incomplete hydration, do not appear to give a full explanation of the reasons behind the problems with regard to dimensional stability. The background to the present invention is rather the idea that the dimensional changes are linked to phase transformations of hydrate. The statement, which is not to be seen as restrictive for the invention, means that calcium aluminate, when it begins to dissolve on the addition of water, forms a gel which then crystallizes and forms hydrate phases. Due to continued hydration reactions and hydrate transformations, various pure Ca aluminate hydrates such as 10-phase, 8-phase, other less defined hydrate phases or transition phases, and finally 6-phase (katoite) can be present, and in the case of additives containing silicon, Ca-Si aluminate hydrate. 10-phase, 8-phase and 6-phase refer to calcium aluminate phases with 10, 8 or 6 water of crystallization per unit of formula. Phase transformation of the hydrates can lead to dimensional changes, especially expansion, which has been shown by long-term evaluation of cement materials. It has turned out surprisingly to be the case in connection with the present invention that with the addition of a secondary phase containing silicon, preferably ordinary so-called Portland cement (OPC cement with Ca-silicates as main phases) and/or fine crystalline silicon dioxide (which constitutes said first, preferred embodiment of the invention), undesirable phase transformations or changed phase transformation sequences can be avoided in the main, and as a direct consequence of this dimensional changes can be minimized, especially long-term expansion. How the complicated hydration reactions come about in detail is not entirely explained. With the addition of material containing Si, the hydration reactions are modified, leading to dimensionally stable materials.

Surprisingly, it has been found that the positive effects just mentioned on the addition of a secondary phase have an optimum with relatively low addition quantities. The minimum expansion has been attained in this connection when said secondary phase consists of OPC cement and/or fine crystalline silicon dioxide and/or another phase containing Si, preferably in a total content of 1-20 percent by volume and even more preferredly 1-10 percent by volume in the material. Most preferredly, said secondary phase consists of OPC cement in a quantity of 1-5 percent by volume and/or fine crystalline silicon dioxide in a quantity of 1-5 percent by volume. Reference is also made in this context to the examples in this specification.

It has also turned out surprisingly that conventional filler particles providing hardness, e.g. in the form of hard Al₂O₃ particles, can be avoided entirely in the material, or that their use can be minimized, in that hardness is controlled primarily by the hydrate developed. Hydrate transformations are the primary cause of dimensional changes with time, in particular long-term changes. The expansion-compensating additives according to the invention act here on the cement phase, without the influence of any hardness-providing filler particles which may be present. Being able to avoid or minimize the use of hardness-providing filler particles is also due to the fact that any unreacted cement remaining - which was previously considered serious from the expansion viewpoint- only has a slight effect on the expansion. It has been found in connection with the invention that unreacted cement instead works positively, as an in-situ filler material, which contributes to the desired hardness of the material.

According to one embodiment of the invention, however, the raw compact, and thus the finished ceramic material, can contain ballast material, which does not take part in the chemical reactions between the binding phase and the hydration liquid, but which is present as a solid phase in the finished ceramic product. According to one aspect of the invention, the raw compact can therefore contain up to 50 percent by volume of ballast material. This ballast material can for example be of the type described in SE 463 493 and SE 502 987, i.e. fibres of metal, carbon, glass or organic material etc., or continuous crystals, so-called whiskers, of e.g. SiC, Si₃N₄ and/or Al₂O₃.

According to another embodiment of the invention, due to additions of aggregates (filler particles) of a given geometry/shape, porosity and/or softness, the dimensional stability of binding agent systems of interest can be monitored precisely and justified to desired levels, often to low levels or to no dimensional change at all. The situation for the cement system CaO-Al₂O₃-(SiO₂)-H₂O, which can be used to advantage as a base material for dental filling material, is described in greater detail below, but the invention relates generally to ceramic binding agent systems in which dimensional stability is critical.

By selecting aggregates (filler particles) in binding agent systems according to the present invention with a specific geometry and porosity, the binding conditions between the binding phase and aggregates can be influenced positively, like the dimensional stability. Porous aggregates and other expansion- or shrinkage-compensating additives thus contribute to the possibilities of being able to justify dimensional changes to a desired level by acting as "expansion vessels".

The function of porous aggregates according to the present invention is thus, with the retention of a high given content of filler particles, to increase the contact surface with the cement phase and distribute this on a smaller propagation area. The expansion which derives from the cement phase is taken up primarily by the porous filler particle in that the cement is given the opportunity to expand inside this. Porous aggregates can consist advantageously of inert ceramic materials such as aluminium oxide, zirconium oxide, titanium oxide or zinc oxide or another oxide or a combination of oxides. The porosity can be present as open or closed porosity or in a combination. In the normal case, the porous particle or aggregate has an open porosity of 20-60 %, preferably 30-50 %. An aggregate size is chosen which is optimally suited to the rupture strength of the materials, but often it has a diameter of less than 20 µm, preferably 5-15 µm. Small porous aggregates or particles contribute in materials of immediate interest to finer surfaces (lower Rₐ-values) than solid particles of a corresponding size. The pore openings in the aggregates are suited to the penetration capacity of the binding agents. The pore openings are advantageously less than 5 µm, preferably 0.1-5 µm and even more preferredly 1-3 µm.

Porous aggregates or particles of the above named oxides are produced preferably by sintering of fine-grained powder, but not at temperatures too high for the aggregates or particles to be kept porous. Aluminium oxide, for example, is best sintered at around 1500-1600°C. The sintering process is controlled to the desired diameter, porosity and size of pores. Alternatively, the porous aggregates or particles can be produced by mixing fine-grained oxide powder with an agent, e.g. starch, which is made to evaporate so that pores are formed. The material is freeze-granulated by being sprayed and frozen.

In a special case, to be able to take up inner stresses caused by dimensional changes in the binding phase, aggregates with a very high closed porosity can be used, which break in the event of high inner stress and provide internal expansion space. The content of these highly porous particles is limited to a maximum of 5 percent by volume of the binding agent phases. Highly porous microspheres of glass can be used in this case. The highly porous materials are added to the cement mixture in the final stage of the mixing operation to avoid being ground down. In another special case, a very soft particle is chosen as an extra additive, which particle can take up stresses by having an E-modulus lower than that of the binding phase. Various soft polymers, e.g. plastic balls, or hydrate can be used here. When using plastic balls, which are very small, these may also have holes in the middle for further deformability.

According to an aspect of the invention, it has also been found that the dimensional stability of the material can be increased by causing the constituent components to have a highly fine granularity. This also applies to strength aspects. The theory in that case is that particles that are too large have a tendency to lie constricted in the structure, with different accompanying attributes in different directions. According to this aspect of the invention, a fine-grained, finely divided mixture of binding agent raw materials is therefore used, which gives a fine homogeneous microstructure. Small propagation areas for the constituent phases reduce the inner mechanical stress between the phases, and provide a better opportunity to compensate for the internal expansion which can take place in the event of changes of phases, such as continued reaction with the surroundings or phase transformations. The size which can be permitted depends on the level of strength desired, but the grain size should typically lie with a distribution over 0.5-10 µm. The calcium aluminate is caused by grinding for 24-72 hours in the presence of a non-polar liquid to have a grain size in the main of around 2-8 µm, preferably 3-4 µm or around 3 µm. OPC cement, if this is used, is caused by grinding in a corresponding manner, possibly at the same time, to have a grain size in the main of around 4-8 µm, preferably 5-7 µm or around 6 µm. Once grinding has been completed, the non-polar liquid is evaporated from the powder mixture. Fine-grained silicon dioxide, if such is used, would have an even smaller grain size, preferably in the order of magnitude of less than 100 nm, and even more preferredly around 10-50 nm, e.g. around 15 nm, which type of silicon dioxide can be purchased for example as a commercial product, separated in electrostatic filters in the production of silicon.

### COMPACTION

The solid constituent parts according to the above are mixed well, at best in the presence of a non-polar liquid, e.g. petroleum ether, acetone or isopropanol, whereupon this non-polar liquid is evaporated from the mixture. The powder mixture is granulated using traditional methods to increase the flow on pressing. The powder mixture, containing expansion-compensating additives and any ballast material, is then compacted to a closely connected raw compact according to the invention. This compaction takes place at high pressure but a low temperature, preferably room temperature, which means that no sintering reactions take place during compaction. Optimization of the degree ofcompactness is important, since it influences properties such as the durability of the raw compact and the strength of the ceramic material in a positive direction, at the same time as it influences the workability of the raw compact on application in a cavity in a negative direction.

According to a previously known technique (SE 463 493), the raw compact is compacted by cold isostatic compaction (CIP), a powder body being arranged in an impermeable casing, which is exposed to outer pressure in a volume of liquid surrounding the casing. The pressure is stated to exceed 200 MPa, at best 250 MPa minimum. According to the present invention, however, the compaction procedure can be simplified considerably in that it can be carried out as simple mechanical tablet pressing, the raw compacts being pressed in tablet form, one by one, in a mechanical tablet press of a conventional type. That this is possible is due to the fact that the raw compacts according to the present invention are relatively small, which is discussed further below. The size of the raw compacts being relatively small, only a small pressure drop occurs in connection with compaction, which means that simple tablet pressing or tablet punching is sufficient to achieve the desired degrees of compactness. The required degree of compactness in this connection is 55-67 percent by volume solid phase. The degree of compactness is preferably 57-63 percent by volume solid phase and even more preferredly 58-61 percent by volume solid phase, the optimum degree of compactness depending on the size of the raw compact, It is to be noted that these degrees of compactness can in certain cases refer to weighted mean values for constituent phases. Tablet pressing is best executed at a pressure of 40 -150 MPa, preferably 70 -110 MPa depending on the table size. The raw compact obtained has a strength (compressive strength) of 0.3 - 5 MPa, preferably 0.5 - 2 MPa, with good edge strength. However, it is not excluded according to the invention that cold isostatic compaction, as described in SE 463 493, can be used in certain cases.

Thanks to tablet pressing to the stated degrees of compactness, a hardness/strength is obtained in the finished ceramic product which is approx. 30-40 % higher than the strength which can be achieved according to the suspension method as described in SE 502 987. At the same time, good workability of the raw compacts is obtained, which workability is better than that which can be achieved with the cold-isostatically compacted raw compacts according to SE 463 493.

According to one aspect of the invention, the raw compact has a largest dimension of 8 mm maximum and a smallest dimension of 0.3 mm minimum, its diameter or width being 1-8 mm, preferably 2-5 mm, and its height being 0.3-5 mm, preferably 0.5-4 mm. To fill a tooth cavity, a number of raw compacts, e.g. 2-5 compacts, are normally needed here. The shape of the raw compacts can be spherical, cylindrical (generally with broken/ chamfered edges) or any other shape which is suitable for tablet pressing and which at the same time gives good strength, e.g. spherical with a cylindrical middle part or a cylinder with a central cut on the flat sides. A tablet-pressed raw compact with an almost spherical shape is easy to grip with instruments from all directions. High cylindrical raw compacts, which are to be applied (in a package) can easily be gripped using a tool (according to below). Low cylindrical raw compacts with a cut on the top and bottom side can be broken in half and fitted into large approximate fillings. The raw compacts can be produced in a number of standard sizes, a minimum size, e.g. with a height of around 0.5-1.5 mm being able to be used for a topmost layer in the tooth filling. This is then easy to pack to a smooth and fine surface without it spreading material to the sides.

According to another aspect of the invention, the shape of the raw compacts can be adapted to a dental drill, so that they fit perfectly into the cavity to be filled. This is applicable to large fillings above all. The tooth cavity is drilled out here using a dental drill with a shape and diameter that match a suitable size of raw compact. The tooth cavity is pretreated and the moistened raw compact inserted directly into the matching hole in the tooth. The raw compact is compacted and packed further into the cavity. Even large fillings, e.g. when a tooth wall is missing, so-called approximate fillings, can be executed in a corresponding manner, the raw compact also being packed against a matrix strip. The advantage of this method is that the strength can be increased further in that the raw compact remains intact right up to being finally packed against the cavity wall or matrix strip. Material wastage is also reduced. In these cases, it is advantageous to use the upper part in the compaction interval, i.e. 61-67 percent by volume solid phase, more preferredly 63-67 percent by volume solid phase.

According to an alternative embodiment, the possibilities of an individualized filling for each cavity are improved further. Here a negative impression is made of the cavity in a suitable impression material, preferably A-silicone mass, whereupon a positive model of the tooth and cavity is produced. Into this die and also exact model of the tooth cavity a raw compact is compacted, which is then steeped in hydration liquid as below, and then applied directly to the tooth cavity as a completely individualized inlay. Subsequent packing with ensuing break-up of the raw compact is suitably avoided. In these cases, it is advantageous to use the upper part in the compaction interval, i.e. 62-67 percent by volume solid phase, more preferredly 64-67 percent by volume solid phase.

### APPLICATION IN A CAVITY

According to one aspect of the invention, the development of strength in the ceramic material can be speeded up, so that the material can be polished and thus finished quickly, the time taken for each patient being shortened and the patient quickly being able to use the repaired tooth, e.g for eating etc. Contradictorily, satisfactory time can be obtained according to the invention simultaneously for shaping and cutting the material before it is hydrated. This is possible on the one hand thanks to the use of an accelerator for hydration of the material, and on the other to the method used for packing the raw compacts into the cavity.

Regarding the use of an accelerator, it has been found that using something of this kind accelerates hydration, and at the same time gives a higher early strength in the material. The hydration process is however affected very little by the accelerator during the first minutes (approx. 2-3 minutes) after the raw compact has been saturated with the hydration liquid, meaning that shaping and cutting of the material can be carried out under not too great a time pressure. The time for shaping is also advantageously extended in that *one* raw compact is immersed at a time, at least partly, in the hydration liquid for at least 5-15 seconds, preferably at least 10 seconds and up to 30 seconds. The liquid is permitted here to be absorbed by capillary forces acting in the raw compact, the raw compact preferably being gripped by means of an insertion instrument in connection with its being immersed in the liquid. The amount of liquid absorbed is automatically at least 90-95 % of that required for complete hydration, meaning that the raw compact takes up 15-22 % of liquid calculated on the amount of powder depending on the degree of compactness. That this is the case is due to the fact that the degree of compactness of the raw compact has been optimized, so that due to capillary forces it absorbs 90-95 % of the required quantity of liquid. Such a quantity of liquid gives good compactability. An improved wetting capacity is obtained preferably by preconditioning of the powder mixture and/or of the raw compact at temperatures exceeding 150°C. Any remaining superficial liquid (visible to the eye) on the raw compact is then dried off, e.g. by bringing the saturated raw compact quite quickly into contact with a moistened serviette. Complete hydration then takes place in the cavity, in connection with saliva secretion during hardening of the material. The raw compact can also be saturated in liquid, dried off against a moistened serviette and then applied to the cavity using an instrument

According to this preferred embodiment of the invention, a first raw compact is thus caused to be saturated by the liquid, in order then to be packed into a cavity, preferably by means of a filling tool, whereupon a second raw compact is caused to be saturated by the liquid, in order then to be packed into said cavity, preferably by means of the same filling tool. Following this, any further raw compacts are caused to be saturated by the liquid, in order then to be packed into the cavity, preferably by means of the same filling tool, at best until the cavity is filled with moist raw compacts packed together, which are then permitted to harden to produce the chemically bound ceramic material. Thanks to the raw compacts being saturated one at a time with hydration liquid, hydration thus commences sequentially for the individual raw compacts, as these are wetted and packed in the cavity. Compared with the method shown in SE 463 493 and in SE 502 987, in which a whole raw pressed cake is wetted, in order then to be broken into pieces and packed in the cavity, a substantial amount of time is thus gained for the moulding. The last material in the saturated raw pressed cake according to previously known techniques will of course have time to begin to hydrate even before it is packed into the cavity and packed together there, possibly resulting in the worst case in uneven packing of the material in the cavity, with the attendant impairment of quality. According to the present invention, however, the material hydrates as it is packed in the cavity. If an accelerator is used, the hydration reaction is accelerated here also, according to the above, after the initial 2-3 minutes, so that a very strong and fast-hardening ceramic material is obtained. The strength can be developed functionally after just 20-60 minutes.

The accelerator preferably consists of a salt of an alkali metal, which is dissolved in the hydration liquid (normally water). Most preferredly, a salt of lithium is used, e.g. lithium chloride or lithium carbonate. The content can be 0.1-1 g/l, preferably at least 0.2 g/l, even more preferredly at least 0.4 g/l and even more preferredly at least 0.8 g/l of water. The higher the content, the quicker the hydration and the greater the strength obtained in the material. Thanks to the sequential wetting and packing, a high content of accelerator can be used without a shortage of time occurring for the application and packing of the material into the cavity. Alternatively, the accelerator, the salt of alkali metal, can be mixed in solid form into the powder mixture before this is compacted. In this connection, the content of accelerator can consist of 0.1-0.5 per mil by volume, preferably 0.2-0.3 per mil by volume in the powder mixture. Since the accelerator is present in this manner in solid form, a further small but perhaps important time gain can be achieved in that the dissolution of the salt following saturation of the raw compact by hydration liquid takes up further time.

When the cavity has been filled with compressed raw compacts, and any top layer formed of one or more thin raw compacts, according to the above, final packing and the removal of any surplus liquid in situ is carried out by means of a compacting tool. Normally there will not be any surplus of hydration liquid, due to the fact that the degree of compactness/ porosity of the raw compact has been optimized so that the quantity of liquid which is automatically absorbed by the capillary forces in the raw compact corresponds to the required quantity of liquid for moulding and initial hydration. However, other liquid may have ended up on the material in connection with its packing into the cavity, e.g. saliva, which is why it is expedient to use a demoistening compaction device/tool for the final compaction. The demoistening operating part of the tool consists here of a hard, porous material, into which any surplus liquid is absorbed at the same time as the tool compresses the raw compact further into the cavity.

Following final packing, subsequent polishing, e.g. grinding of a free surface of the chemically bound ceramic material facing towards the oral cavity can be carried out, preferably within 3-10 minutes, even more preferredly within 3-7 minutes after compaction of the moist raw compacts has been completed.

### TOOL FOR INSERTION AND FINAL COMPACTION

A suitable tool for insertion of a raw compact into a cavity is described in the following. The term insertion refers to a raw compact being gripped, held, introduced into the cavity that is intended to be filled and left/attached there.

The insertion tool comprises a cylindrical sheath and preferably a nozzle or head with an internal diameter adapted according to a diameter of a given raw compact. The sheath has an open first short end for receiving the raw compact, and a piston arranged in the sheath, which piston is displaceable in the axial direction of the sheath, for transferring the raw compact to the cavity.

The tool may e.g. exhibit a minimum internal diameter of the cylindrical sheath, or nozzle, which is less than the diameter of the given raw compact, the sheath, at its first short end or nozzle, having an internal chamfer of approx. 20°, the largest diameter of which exceeds the diameter of the given raw compact.

A preferred tool is also adapted, when not gripping a raw compact, to grip instead and hold a compaction body, which has a diameter adapted to the internal diameter of the sheath or nozzle and consists of a relatively hard, porous material, due to which the tool forms a demoistening compaction device for compaction in the cavity of one or more raw compacts saturated with said liquid. Due to this, the same tool can thus be used for insertion of the raw compacts into the cavity, and for the final demoistening packing together (compaction) of the raw compacts in the cavity.

A suitable compaction body for use in the method according to the invention consists of a material in the group consisting of porous ceramic materials, porous polymer materials, porous metal materials and porous wood materials, preferably a material consisting of a hardwood material, The material for the compaction body should have pores with a smaller diameter than the powder grains in the material that is to be compressed. It has been found surprisingly that beech wood functions excellently as a material for the compaction body. The compaction body at beat has a diameter that corresponds to the most commonly used diameter of raw compacts, preferably 1-8 mm, even more preferredly 2-5 mm. By means of the compaction device provided with the compaction body, an advantageously combined pressing and suction effect is obtained on the raw compacts packed into the cavity.

### BRIEF DESCRIPTION OF DRAWINGS

Some of the aspects according to the invention will be described further below with reference to the enclosed figures, of which:
- Figs. 1a-c: show some conceivable embodiments of the raw compact according to the invention;
- Figs. 2a-c: show, from the side or from above, a tooth that is drilled and provided with one or more raw compacts according to the invention;
- Figs. 3a-b: show a model/die of a tooth with a cavity, and a raw compact which has been moulded in the model/die;
- Fig. 4: shows strength as a function of time for some varying concentrations of accelerator in the hydration liquid;
- Fig. 5: shows a view in perspective of a tool, i.e. an insertion instrument, including raw compact, suitable for use in the method of the insertion;
- Fig. 6: shows a side view of the tool in Fig. 5 in cross-section, including raw compact;
- Fig. 7: shows the head of the tool according to Fig. 6, in cross-section;
- Fig. 8: shows the tip of the head according to Fig. 7, in section, including piston and raw compact;
- Fig. 9: shows a side view of two variants of a head for alternative embodiments of the tool;
- Fig. 10: shows a side view of yet another variant ofa head for an alternative embodiment of the tool;
- Fig. 11: shows a side view of a head for a tool for use in the method according to the invention, which grips a compacting body; and
- Fig. 12: shows expansion as a function of time for a ceramic material produced according to the invention.

### DETAILED DESCRIPTION OF THE INVENTION

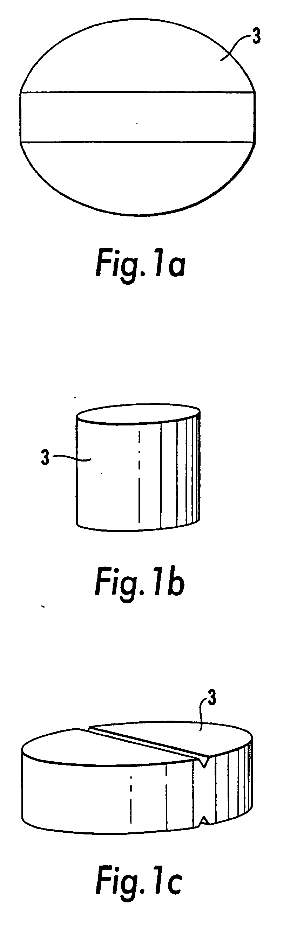

Figs. 1a-c show some conceivable forms of a raw compact according to the invention, to be precise a spherical shape with a cylindrical middle part, a high cylindrical form or a low cylindrical form with a cut for simplified division if so desired.

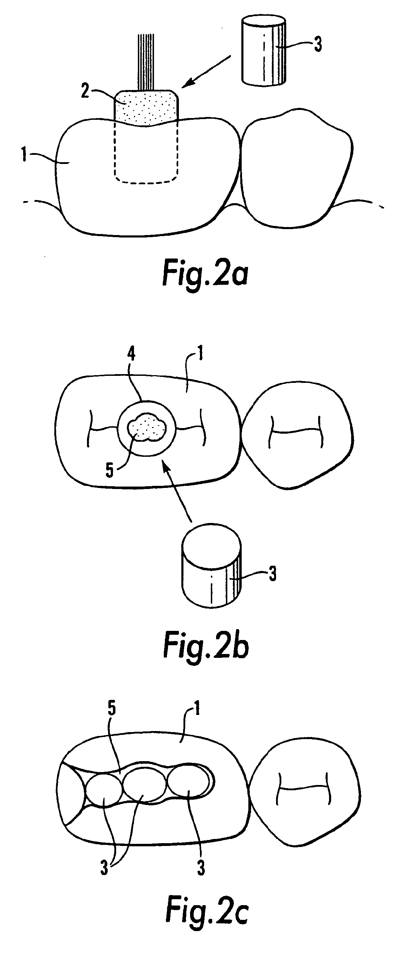

Figs. 2a-b show how a tooth 1 is drilled out with a drill 2 of a selected diameter, whereupon a cylindrical raw compact 3 of a corresponding diameter can be inserted into the cavity 4 formed in the tooth 1. Fig. 2b also shows the extent of an original attack of decay 5. Fig. 2c shows how several raw compacts 3 can be inserted into a large cavity 5.

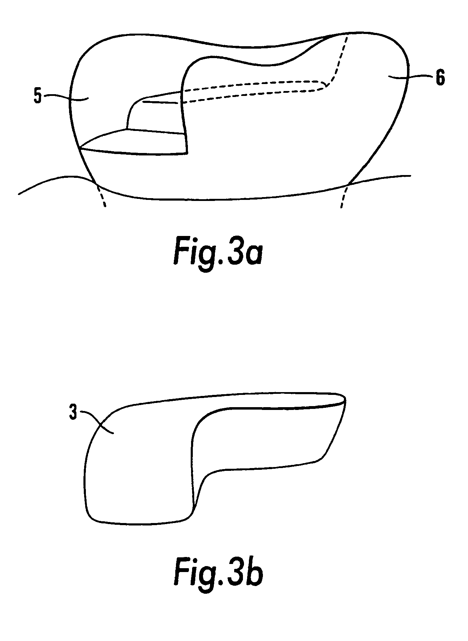

Fig. 3a shows a positive model 6 of a tooth, with a cavity 5. This model 6 has been produced from a negative impression (not shown) of an individual tooth with cavity. In a compaction stage for a raw compact, the model 6 is used as a mould or die, a raw compact 3 (Fig. 3b) being obtained that is perfectly suited to the individual tooth with its cavity. This raw compact 3 is placed following moistening into the cavity in the tooth and then preferably not packed any further.

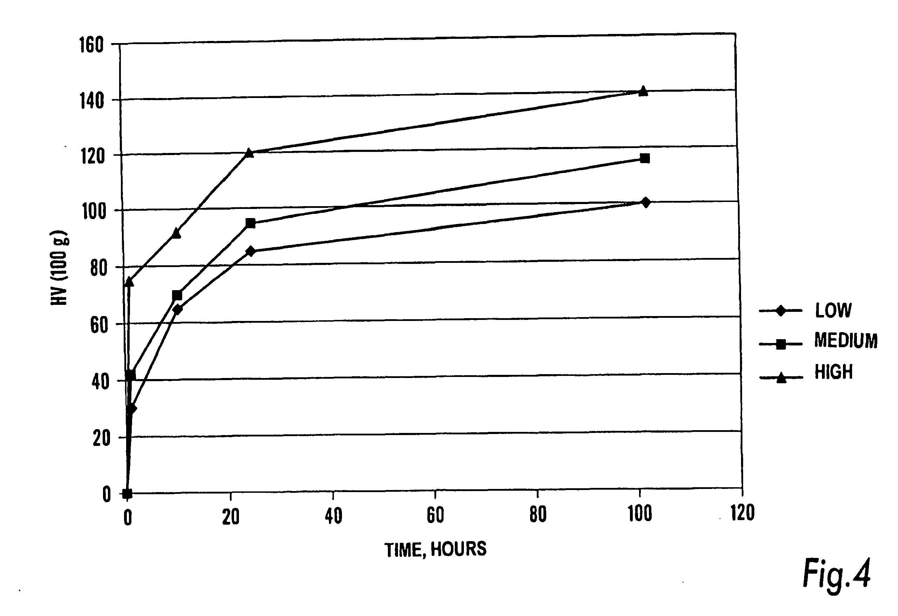

Fig. 4 shows the hardness achieved (measured as HV 100g) in the ceramic material formed as a function of time with some varying concentrations of accelerator, in this case lithium chloride, in the hydration liquid, which consists in this case of water. As is evident, the hardness is developed more quickly and to higher final values the higher the concentration of accelerator used. The levels low, medium and high refer to 1 10⁻⁴ g, 2 10⁻⁴ g and 3 10⁻⁴ g respectively of lithium salt/g of formed product.

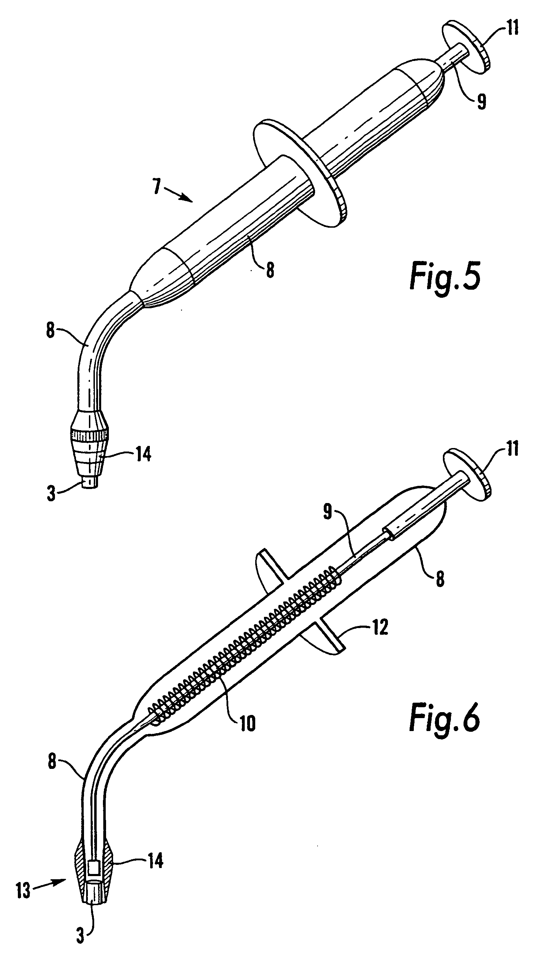

Figs. 5 and 6 show a tool 7, which can have an external design that can be the same as for conventional insertion tools and can be produced in plastic or metal, preferably steel. The tool comprises a cylindrical sheath 8 and a piston 9 arranged in the sheath, which piston is displaceable in the axial direction of the sheath. The piston 9 is enclosed in a coil spring 10, which prevents the piston being led too far through the sheath 8. The tool 7, more precisely the piston 9, is also provided with a pressing surface 11 for pressing by a finger, e.g. the thumb, of the user. A projecting dolly 12 is best arranged on the sheath to be gripped by two other fingers, e.g. the index finger and middle finger. The sheath 8 in the embodiment shown is bent to achieve the best means of access to the oral cavity. The tip 13 of the tool is best formed by a head 14, which can be detachable, preferably threaded, so that the tool can be used for raw compacts of a number of different dimensions.

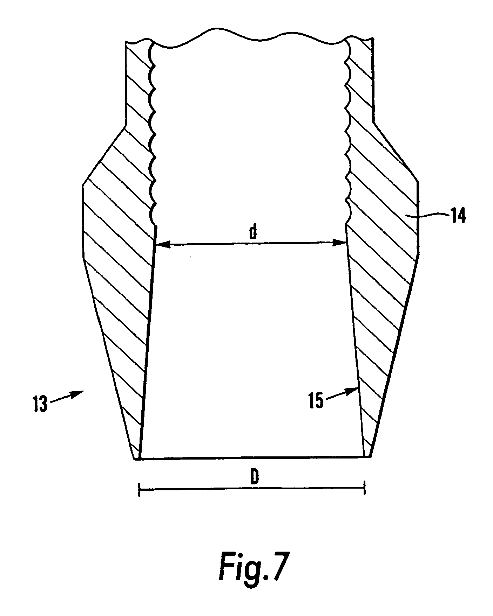

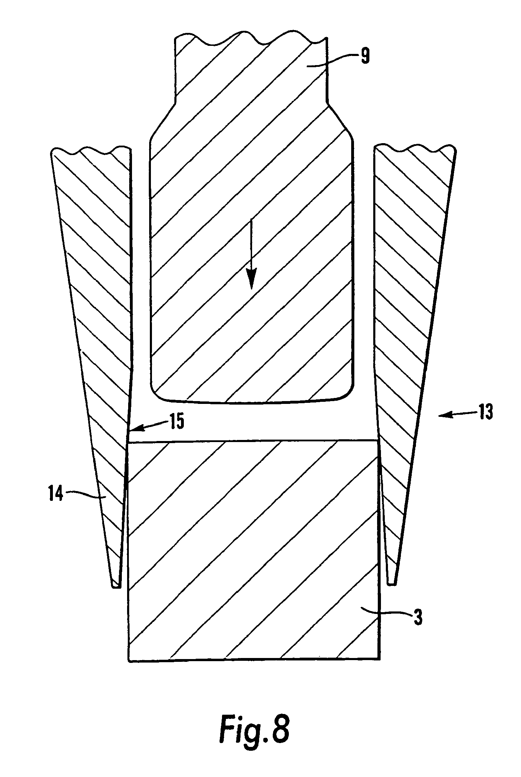

So that it is not too difficult to grip the raw compact, the sheath 8 has an internal chamfer 15 by the head 14, the greatest diameter D of which chamfer exceeds the diameter of a raw compact 3 of a given size and the minimum diameter d of which is less than the diameter of the given raw compact 3, according to Figs. 7 and 8. The chamfer 15 suitably has an angle of around 20° relative to the centre line of the head 14. Due to this, the raw compact can be introduced easily into the tool, and nevertheless held steadily in place in the same. Fig. 8 shows how the raw compact is held firmly by means of the chamfer 15, it also being indicated how the piston 9 can be displaced so that it pushes the raw compact 3 out of the head 14.

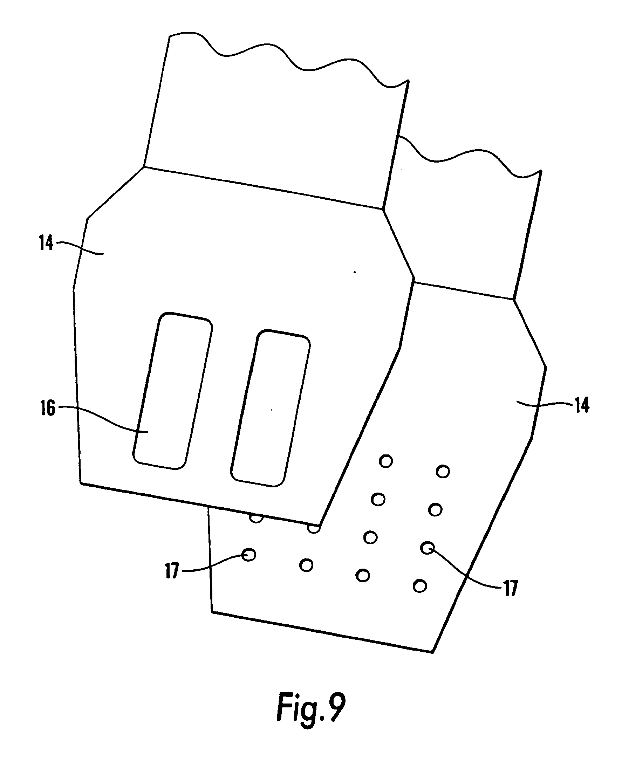

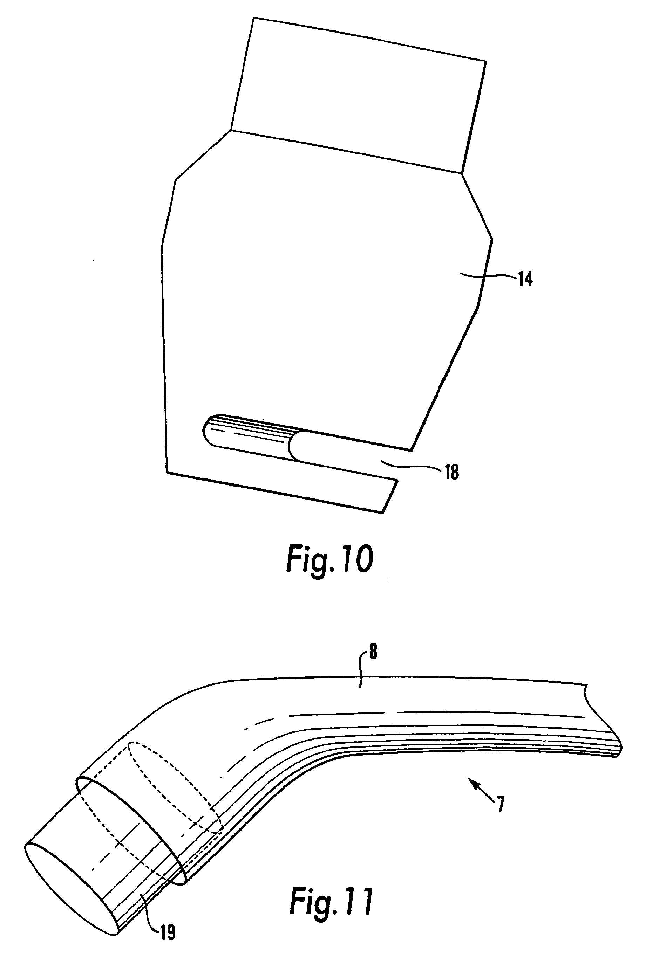

According to Fig. 9, the tool, or more precisely the head 14, can be provided with perforations, e.g. in the form of slots 16 or holes 17, with a view to increasing the uptake of hydration liquid when the gripped raw compact 3 is partly immersed in the liquid. A number of slots 16 or holes 17 are here arranged around the circumference of the head 14. An alternative, according to Fig. 10, is to provide the head 14 with a recess 18 in the wall, which suitably extends a little way around the circumference of the head 14 and is arranged a little way, e.g. 3 mm, up from the short end of the head.

The embodiments according to Fig. 9 and 10 are only conceivable variants. In normal cases no perforation or recess is required, since the capillary action of the raw compact is so good that the necessary quantity of liquid is absorbed even when only the lower, projecting part of the raw compact 3 is immersed in the liquid.

Fig. 11 shows how the tool 7 with the head 14 can also grip a compaction body 19, for final compaction of the raw compacts in the cavity. This compaction body 19 consists according to the previous description of a hard, porous material, and has a diameter that is adapted to be gripped by the tool 7.

### EXAMPLE 1

A series of experiments was performed to study the effect on expansion, in particular long-term expansion, of various expansion-compensating additives.

### Description of raw materials:

Calcium aluminate of the phases CaO.Al₂O₃ and CaO.2Al₂O₃ forming part of e.g. Ca-aluminate cement (Alcoa or LaFarge), standard cement (Cementa), fine-grained silicon dioxide (Aldrich) and glass spheres (Sil-cell, Stauss GmbH). Al₂O₃ (Sumitomo, AKP 30), ZrO₂ (3-mol % Y₂O₃) from Toyo Soda.

Porous particles, produced in-house from fine-grained Al oxide (Sumitomo, AKP 30) (aggregate diameter approx. 15 micrometres)

### The examples under a) - h) describe

a) calcium aluminate's long-term expansion with completely hydrated aluminate without additives, but with hardness-providing filler particles (reference)
b) effect of fine granularity of raw cement materials
c) effect of secondary phase, OPC cement
d) effect of secondary phase, fine-grained Si oxide
e) effect of porous aggregate on b)
f) effect of porous aggregate on c)
g) effect ofa combination of OPC and fine-grained Si oxide
h) effect of a combination of various additives
i) effect of Si-containing secondary phases on a pure cement system without hardness-providing filler particles
j) effect of hardness-providing filler particles on i)

Calcium aluminates. CaO.Al₂O₃ and CaO.2Al₂O₃, with a molecular ratio of approx. 1:1 are mixed with filler particles and secondary additives (all quantities specified are in relation to the quantity of calcium aluminate) as stated below. When "aluminium oxide" is referred to, without the type of particles being specified, conventional hardness-providing filler particles are meant.
a) Addition of 40 percent by volume aluminium oxide, grinding time 24 h. The cement was ground for 20 h beforehand.
b) Addition of 40 percent by volume aluminium oxide, grinding time 24 h. The cement was ground for 80 h beforehand.
c) Addition of 40 percent by volume aluminium oxide, grinding time 24 h. The cement was ground according to b) above beforehand. 15 percent by volume OPC (ordinary Portland cement/standard cement) was added to the calcium aluminate.
d) Addition of 40 percent by volume aluminium oxide, grinding time 24 h. A secondary phase in the form of 10 percent by volume fine-grained silicon dioxide was added to the ground calcium aluminate according to b) above.
e) Addition of 20 percent by volume aluminium oxide, grinding time 24 h. The cement was ground according to b) above. 20 percent by volume of porous aluminium oxide aggregates(produced in-house) was added only after a grinding time of 20 h.
f) Addition of 20 percent by volume aluminium oxide + 20 percent by volume aluminium oxide as porous particles (aggregates), grinding time 24 h, but the aggregates were only added after 20 h. The cement was ground according to b) above, but with the addition of a secondary phase in the form of 15 percent by volume OPC.
g) Addition of 40 percent by volume aluminium oxide, grinding time 24 h. The cement was ground according to b) above. 5 percent by volume of OPC and 5 percent by volume offine-grained silicon dioxide were added to the calcium aluminate.
h) Addition of 20 percent by volume aluminium oxide + 20 percent by volume aluminium oxide as porous particles, grinding time 24 h, but the aggregates were only added after 20 h. A secondary phase in the form of 5 percent by volume of OPC and 5 percent by volume of fine-grained silicon dioxide and 0.5 percent by volume of glass spheres was added to the calcium aluminate in this case.
i) Addition of secondary phases in the form of 5 percent by volume of OPC and 5 percent by volume of fine-grained silicon dioxide, grinding time 24 h. The cement was ground beforehand for 80 h.
j) Addition of secondary phases in the form of 5 percent by volume of OPC and 5 percent by volume of fine-grained silicon dioxide and hardness-providing filler particles of ZrO₂ of 10 percent by volume, grinding time 24 h. The cement was ground beforehand for 80 h.

The mixtures were ground in a ball mill with inert grinding balls of silicon nitride with a coefficient of fullness of 35 %. Isopropanol was used as a liquid. Following evaporation of the solvent, materials a) - h) were admixed with water, dewatered and tamped with a stopper into holes with a diameter of 4 mm in a container that permitted measurement of the dimensions in an optical microscope. The materials were kept moist at 37°C between test measurements, which were performed continuously up to 180 days.

The results are reported in the table below.

| Sample description | Expansion in % after | | | | |
|---|---|---|---|---|---|
| | 1d | 2d | 80d | 120d | 180d |
| a | 0 | 0.12 | 0.68 | 0.82 | 0.83 |
| b | 0 | 0.22 | 0.41 | 0.48 | 0.48 |
| c | 0 | 0.11 | 0.23 | 0.26 | 0.26 |
| d | 0 | 0.12 | 0.13 | 0.13 | 0.13 |
| e | 0 | 0.15 | 0.18 | 0.21 | 0.21 |
| f-j | all values under 0.10 % | | | | |

| | | | | | |
|---|---|---|---|---|---|
| Error margin for measurements +-0.02 %. | | | | | |

It is evident from the results that expansion stagnates after approx. 100 days. For the very dimensionally stable materials (expansion under 0.15 % including error margins) no clear point in time for stagnation can be deduced. It is also evident that
- Only an increased grinding time (b) in relation to reference (a) almost halves long-term expansion.
- When a secondary phase in the form of OPC cement in a quantity of 15 percent by volume is also added (c), a further virtual halving of long-term expansion is achieved in relation to (b).
- Long-term expansion is further reduced by a secondary phase in the form of fine-grained silicon dioxide in a quantity of 10 percent by volume (d).
- Improved (reduced) long-term expansion in relation to (b) is also achieved using porous aggregates of aluminium oxide in a quantity of 20 percent by volume (e).
- Extremely low expansion was achieved when using porous particles and a secondary phase of OPC cement in combination.
- Extremely low expansion was achieved when using a secondary phase of both OPC cement and fine-grained silicon dioxide, in combination.
- Extremely low expansion was achieved using porous particles, a secondary phase of both OPC cement and fine-grained silicon dioxide and glass spheres in combination.
- Extremely low expansion was achieved for the pure cement system without hardness-providing filler particles when using only a low content of Si-containing additives.
- Extremely low expansion was achieved for the Ca-aluminate system with low quantities (10 percent by volume) of hardness-providing filler particles, using only Si-containing additives for expansion compensation.

The materials which have extremely low expansion (<0.10 %) have, apart from the expansion properties, a general feature profile which matches the corresponding cement system without additives. These materials have a compressive strength of 170-200 MPa, hardness ofH (Vickers 100g) = 110-130 and an extremely high acid resistance.

### EXAMPLE 2

### Description of raw materials:

Calcium aluminate of the phases CaO.Al₂O₃ and CaO.2Al₂O₃ forming part of e.g. Ca-aluminate cement (Alcoa or LaFarge), standard cement (Cementa), silicon dioxide (Aldrich) and an inert oxide, ZrO₂ (Aldrich).

### Mixing:

The cement phases were ground together for approx. 80 h, whereupon the oxides were added in a final mixing operation of approx. 22 h. Finally, the grinding liquid, consisting of 2-propanol, was evaporated.

### Pressing:

The powder mixture was pressed into small tablets at a pressure of approx. 90 MPa in a Korsch tablet punching machine. Tablet diameter 3.00 mm and height 3.00 mm with approx. 20° chamfer 0.35 mm in from the flat sides.

### Conditioning:

The tablets were heat-treated at 400°C for 4 h.

### Insertion and compaction in the test chamber:

Water with alkali salt was added to the tablets one at a time. The tablets were transferred to the cavity in a special holder and compacted with an ordinary stopper initially, to be tamped finally with a porous compacting device (according to Fig.11). They were then kept in the test bodies at 37°C in a humid environnment.

### Result:

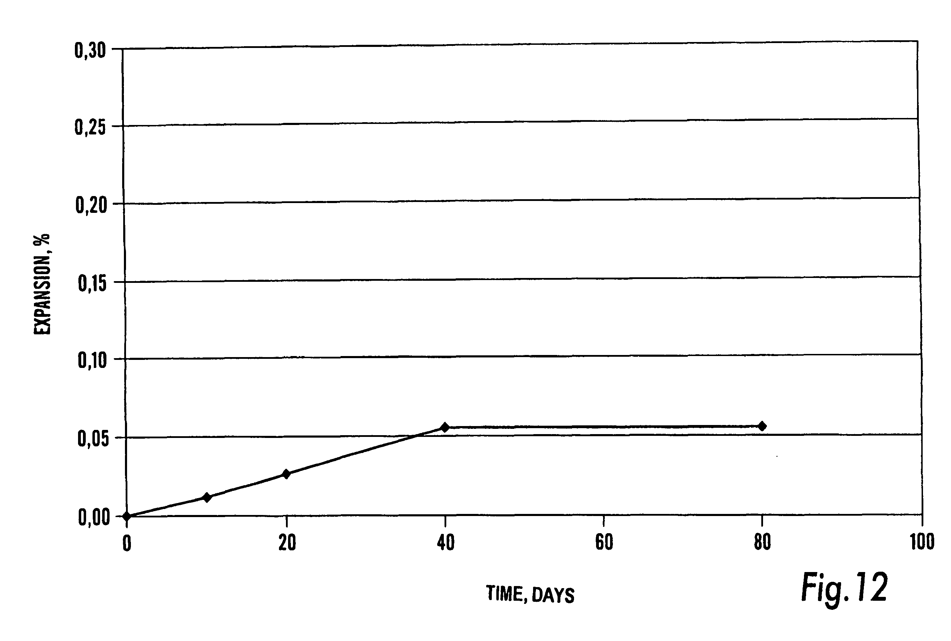

Fig. 12 shows the resulting expansion as a function of time for the ceramic material formed. It is confirmed that the expansion values achieved are very low and that expansion levels out after 40 days.

The invention is not restricted to the embodiments described, but can be varied within the slope of the claims. Thus it is understood for example that the invention can also be used in connection with non-dental applications, i.e. in connection with the production of chemically bound ceramic materials for other purposes. A conceivable application area is e.g. a carrier material (substrate) for electronic circuits and a substrate in micromechanics, The product can consist here for example of a thin plate with a diameter of around 2.7 cm, for use as a carrier material for electronic circuits, as a carrier material in micromechanical applications, as a carrier material for biosensors or as a carrier material for optical fibres for producing circuit boards, biosensors or the like.

## Claims

1. Raw compact (3) comprising a binding phase of one or more powdered binding agents, which raw compact has the capacity following saturation with a hydration liquid reacting with the binding agents to form a chemically bound ceramic material, which raw compact (3) also comprises one or more expansion-compensating additives adapted to give the material dimensionally stable long-term properties, wherein said additives have been selected from the group consisting of one or more additives in the group which consists of porous particles or porous aggregates, soft particles which have an E-modulus which is lower than the E-modulus of the binding phase, and a secondary phase, which secondary phase reacts with the binding phase, wherein the expansion-compensating additives are present in an amount of approximately 1-20 percent by volume of the raw compact, **characterised in that** the raw compact has a degree of compactness of 55-67 percent by volume solid phase.

2. Raw compact according to claim 1, **characterised in that** the raw compact (3) is substantially free of hardness-providing filler particles.

3. Raw compact according to claim 1, **characterised in that** the raw compact (3) comprises up to 50 percent by volume ballast material.

4. Raw compact according to any of the preceding claims, **characterised in that** said binding phase consists at least substantially of calcium aluminate cement.

5. Raw compact according to any of the preceding claims, **characterised in that** said additives consist at least substantially of a secondary phase, said secondary phase preferably consisting of OPC cement and/or fine crystalline silicon dioxide and/or another Si-containing phase, preferably in a total quantity of 1-20 percent by volume and even more preferredly 1-10 percent by volume in the raw compact.

6. Raw compact according to claim 5, **characterised in that** said secondary phase consists of OPC cement in a quantity of 1-5 percent by volume and/or fine crystalline silicon dioxide in a quantity of 1-5 percent by volume.

7. Raw compact according to any of claims 1-6, **characterised in that** said additives consist at least substantially of porous particles or porous aggregates, which consist at least substantially of oxides of Al, Zr, Ti, Si, or Zn and Sn, these preferably having a diameter of 2-30 µm, even more preferredly 5-15 µm, an open porosity of 20-60 %, preferably 30-50 %, and the pore openings in the particles/aggregates being less than 5 µm, preferably 0.1-5 µm and even more preferredly 1-3 µm.

8. Raw compact according to any of claims 1-6, **characterised in that** said additives at least substantially consist of porous particles, which porous particles consist at least substantially of microspheres with a high closed porosity, which microspheres are preferably of glass and have a porosity which exceeds 50 %, preferably exceeds 80 %, and are present in quantities of less than 2 percent by volume of the raw compact.

9. Raw compact according to any of the preceding claims, **characterised in that** said raw compact (3) has a degree of compactness of 57-63 percent by volume solid phase, preferably 58-61 percent by volume solid phase.

10. Raw compact according to any of the preceding claims, **characterised in that** said raw compact (3) also comprises an accelerator for accelerated reaction between said binding agents and the hydration liquid, said accelerator consisting preferably of a salt of an alkali metal, in a quantity of 0.1-0.5 per mil by volume, preferably 0.2-0.3 per mil by volume, calculated on the solid content.

11. Raw compact according to any of the preceding claims, **characterised in that** said binding agents substantially have a grain size of 2-8 µm, preferably 3-4 µm.

12. Raw compact according to any of the preceding claims, **characterised in that** said raw compact (3) has a largest dimension of 8 mm maximum and a smallest dimension of 0.3 mm minimum, its diameter or width being 1-8 mm, preferably 2-5 mm, and its height being 0.3-5 mm, preferably 0.5-4 mm.

13. Method of producing a raw compact as defined in claims 1-12, comprising the steps of
- mixing a binding phase of one or more powdered binding agents, which raw compact (3) has the capacity following saturation with a hydration liquid reacting with the binding agents to form a chemically bound ceramic material, one or more expansion-compensating additives adapted to give the material dimensionally stable long-term properties, wherein said additives have been selected from the group consisting of one or more additives in the group which consists of porous particles or porous aggregates, soft particles which have an E-modulus which is lower than the E-modulus of the binding phase, and a secondary phase, which secondary phase reacts with the binding phase, wherein the expansion-compensating additives are present in an amount of approximately 1-20 percent by volume of the raw compact;
- forming a powder body of said powdered material and additives;
- exposing the powder body to a pressure 40-150 MPa and a temperature from room temperature to at least 150°C, such that a closely connected raw compact (3), having a compaction degree of 55-67 percent by volume solid phase, is obtained without sintering reactions.

14. Method according to claim 13, **characterised in that** the raw compact (3) is substantially free of hardness-providing filler particles.

15. Method according to claim 13, **characterised in that** the raw compact (3) comprises up to 50 percent by volume ballast material.

16. Method according to any of claims 13-15, **characterised in that** said binding phase consists at least substantially of calcium aluminate cement.

17. Method according to any of claims 13-16, **characterised in that** said raw compact (3) is caused to have a degree of compactness of 57-63 percent by volume solid phase, preferably 58-61 percent by volume solid phase.

18. Method according to any of claims 13-17, **characterised in that** said additives consist at least substantially of a secondary phase, said secondary phase preferably consisting of OPC cement and/or fine crystalline silicon dioxide and/or another Si-containing phase, preferably in a total quantity of 1-20 percent by volume and even more preferably 1-10 percent by volume in the raw compact.

19. Method according to claim 18, **characterised in that** said secondary phase consists of OPC cement in a quantity of 1-5 percent by volume and/or fine crystalline silicon dioxide in a quantity of 1-5 percent by volume.

20. Method according to any of claims 13-19, **characterised in that** said additives consist at least substantially of porous particles or porous aggregates, which consist at least substantially of oxides of Al, Zr, Ti, Si, or Zn and Sn, these preferably having a diameter of 2-30 µm even more preferredly 5-15 µm, an open porosity of 20-60 %, preferably 30-50 %, and the pore openings in the particles/aggregates being less than 5 µm, preferably 0.1-5 µm and even more preferredly 1-3 µm.

21. Method according to any of claims 13-19, **characterised in that** said additives at least substantially consist of porous particles, which porous particles consist at least substantially of microspheres with a high closed porosity, which microspheres are preferably of glass and have a porosity which exceeds 50 %, preferably exceeds 80 %, and are present in quantities of less than 2 percent by volume of the raw compact.

22. Method according to any of claims 13-21, **characterised in that** said raw compact (3) has a degree of compactness of 57-63 percent by volume solid phase, preferably 58-61 percent by volume solid phase.

23. Method according to any of claims 13-22, **characterised in that** said raw compact (3) also comprises an accelerator for accelerated reaction between said binding agents and the hydration liquid, said accelerator consisting preferably of a salt of an alkali metal, in a quantity of 0.1-0.5 per mil by volume, preferably 0.2-0.3 per mil by volume, calculated on the solid content.

24. Method according to any of claims 13-23, **characterised in that** said binding agents substantially have a grain size of 2-8 µm, preferably 3-4 µm.

25. Method according to any of claims 13-24, **characterised in that** said raw compact (3) is made to have a largest dimension of 8 mm maximum and a smallest dimension of 0.3 mm minimum, its diameter or width being 1-8 mm, preferably 2-5 mm, and its height being 0.3-5 mm, preferably 0.5-4 mm.

26. Method according to any of claims 13-25, **characterised in that** the method comprises a tablet pressing stage for each raw compact (3) that is produced, tablet pressing being executed at a pressure of 40-150 MPa, preferably 70-110 MPa.

27. Method according to any of claims 13-26, **characterised in that** said raw compact (3) is formed **in that** said compaction is executed in a model (6) of a cavity (5) into which the raw compact is to be introduced.

28. Method according to any of claims 13-27, **characterised in that** the powder mixture and/or said raw compact (3) is preconditioned at temperatures exceeding 150°C.

29. Cured material based on the raw compact defined in claims 1-12, in hydrated form.

30. Cured material according to claim 29, **characterised in that** said material has a compressive strength of 170-200 MPa.

31. Cured material according to claim 29 or 30, **characterised in that** said material has a Vickers hardness 110-130.

32. Method of producing the cured material defined in claims 29-31, comprising the step of contacting a raw compact defined in claims 1-12 with a hydration liquid.

33. Method according to claim 32, **characterised in that** said hydration liquid is caused to saturate the raw compact (3) **in that** the raw compact is immersed at least partly in the hydration liquid, preferably for at least 5-15 seconds, preferably at least 10 seconds and up to 30 seconds, so that the hydration liquid is permitted to be absorbed by the capillary forces acting in the raw compact, the raw compact preferably being gripped by means of an insertion instrument (7) in connection with its being immersed in the hydration liquid and any remaining superficial hydration liquid on the raw compact then being dried off.

34. Method according to claim 32 or 33, **characterised in that** said hydration liquid preferably contains an accelerator for the reaction between the binding phase and the hydration liquid, said accelerator preferably consisting of a salt of an alkali metal, preferably in a quantity of 0.1-1 g/l of liquid, preferably at least 0.2 g/l, even more preferredly at least 0.4 g/l and most preferredly at least 0.8 g/l of liquid.

35. Method according to claim 33, **characterised in that** a first raw compact (3) is caused to be saturated by the hydration liquid, in order then to be packed into a cavity (5), preferably by means of a filling tool, whereupon a second raw compact (3) is caused to be saturated by the hydration liquid, in order then to be packed into said cavity (5), preferably by means of said filling tool, whereupon any further raw compacts are caused to be saturated by the hydration liquid, in order then to be packed into said cavity, preferably by means of said filling tool, preferably until the cavity has been filled with moist, compressed raw compacts, which are then permitted to harden to said chemically bound ceramic material.

36. Method according to claim 35, **characterised in that** final packing and the removal of any surplus of hydration liquid is executed in situ by means of a compaction device (7) - a tool - the part (19) of which acting against the moist, compacted raw compacts consists of a hard, porous material, into which any surplus of hydration liquid is absorbed, at the same time as the moist raw compacts (3) packed together are compacted further in the cavity (5).

37. Method according to claim 35 or 36, **characterised in that** subsequent polishing of a free surface of the chemically bound ceramic material formed is executed, preferably within 3-10 minutes, even more preferredly within 3-7 minutes after said compaction of the moist raw compacts (3) has been completed.

38. Dental filling material comprising the raw compact according to any of claims 1-12 or the cured material according to claims 29-31.

39. Electronic circuit, biosensor and optical fibre having a carrier of a material comprising the raw compact according to any of claims 1-12 or the cured material according to claims 29-31.

40. Use of the raw compact according to any of claims 1-12 or the cured material according to claims 29-31 as a carrier material in a micromechanical application.

## Patentansprüche

1. Roher Presskörper (3), umfassend eine bindende Phase aus einem oder mehreren pulverförmigen Bindemitteln, wobei der rohe Presskörper die Eigenschaft aufweist, nach Sättigung mit einer Hydratisierungsflüssigkeit mit den Bindemitteln zu reagieren, um ein chemisch gebundenes keramisches Material zu bilden, wobei der rohe Presskörper (3) außerdem ein oder mehrere Expansions-kompensierende Additive umfasst, die geeignet sind, um dem Material dimensionsstabile Langzeiteigenschaften zu verleihen, wobei die Additive ausgewählt worden sind aus der Gruppe bestehend aus einem oder mehreren Additiven in der Gruppe, bestehend aus porösen Teilchen oder porösen Aggregaten, weichen Teilchen, die einen E-Modul aufweisen, der niedriger als der E-Modul der bindenden Phase ist, und einer sekundären Phase, wobei die sekundäre Phase mit der bindenden Phase reagiert, wobei die Expansions-kompensierenden Additive in einer Menge von ungefähr 1 bis 20 Volumenprozent des rohen Presskörpers vorhanden sind, **dadurch gekennzeichnet, dass** der rohe Presskörper einen Verdichtungsgrad von 55 bis 67 Volumenprozent der festen Phase aufweist.

2. Roher Presskörper gemäß Anspruch 1, **dadurch gekennzeichnet, dass** der rohe Presskörper (3) im Wesentlichen frei von Füllstoffteilchen, die Härte bereitstellen, ist.

3. Roher Presskörper gemäß Anspruch 1, **dadurch gekennzeichnet, dass** der rohe Presskörper (3) bis zu 50 Volumenprozent Ballastmaterial umfasst.

4. Roher Presskörper gemäß einem der vorstehenden Ansprüche, **dadurch gekennzeichnet, dass** die bindende Phase mindestens im Wesentlichen aus Calciumaluminatzement besteht.

5. Roher Presskörper gemäß einem der vorstehenden Ansprüche, **dadurch gekennzeichnet, dass** die Additive mindestens im Wesentlichen aus einer sekundären Phase, wobei die sekundäre Phase vorzugsweise aus OPC-Zement und/oder feinem kristallinem Siliciumdioxid und/oder einer anderen Si-haltigen Phase, bevorzugt in einer Gesamtmenge von 1 bis 20 Volumenprozent und noch stärker bevorzugt 1 bis 10 Volumenprozent im rohen Presskörper bestehen.

6. Roher Presskörper gemäß Anspruch 5, **dadurch gekennzeichnet, dass** die sekundäre Phase aus OPC-Zement in einer Menge von 1 bis 5 Volumenprozent und/oder feinem kristallinen Siliciumdioxid in einer Menge von 1 bis 5 Volumenprozent besteht.

7. Roher Presskörper gemäß einem der Ansprüche 1 bis 6, **dadurch gekennzeichnet, dass** die Additive mindestens im Wesentlichen aus porösen Teilchen oder porösen Aggregaten bestehen, welche mindestens im Wesentlichen aus Oxiden von Al, Zr, Ti, Si oder Zn und Sn bestehen, wobei diese vorzugsweise einen Durchmesser von 2 bis 30 µm, noch stärker bevorzugt 5 bis 15 µm, eine offene Porosität von 20 bis 60 %, bevorzugt 30 bis 50 % aufweisen, und wobei die Porenöffnungen in den Teilchen/Aggregaten weniger als 5 µm, bevorzugt 0,1 bis 5 µm und hoch stärker bevorzugt 1 bis 3 µm aufweisen.

8. Roher Presskörper gemäß einem der Ansprüche 1 bis 6, **dadurch gekennzeichnet, dass** die Additive mindestens im Wesentlichen aus porösen Teilchen bestehen, wobei die porösen Teilchen mindestens im Wesentlichen aus Mikrokügelchen mit einer hohen geschlossenen Porosität bestehen, wobei die Mikrokügelchen vorzugsweise aus Glas sind und eine Porosität aufweisen, die 50 % übersteigt, vorzugsweise 80 % übersteigt, und in Mengen von weniger als 2 Volumenprozent des rohen Presskörpers vorhanden sind.

9. Roher Presskörper gemäß einem der vorstehenden Ansprüche, **dadurch gekennzeichnet, dass** der rohe Presskörper (3) einen Verdichtungsgrad von 57 bis 63 Volumenprozent der festen Phase, bevorzugt 58 bis 61 Volumenprozent der festen Phase aufweist.

10. Roher Pulverpressling gemäß einem der vorstehenden Ansprüche, **dadurch gekennzeichnet, dass** der rohe Presskörper (3) außerdem einen Beschleuniger für die beschleunigte Reaktion zwischen den Bindemitteln und der Hydratisierungsflüssigkeit, wobei der Beschleuniger vorzugsweise aus einem Salz eines Alkalimetalls besteht, in einer Menge von 0,1 bis 0,5 Volumenpromille, bevorzugt 0,2 bis 0,3 Volumenpromille, bezogen auf den Feststoffgehalt, umfasst.

11. Roher Presskörper gemäß einem der vorstehenden Ansprüche, **dadurch gekennzeichnet, dass** die Bindemittel im Wesentlichen eine Korngröße von 2 bis 8 µm, bevorzugt 3 bis 4 µm aufweisen.

12. Roher Presskörper gemäß einem der vorstehenden Ansprüche, **dadurch gekennzeichnet, dass** der rohe Presskörper (3) eine größte Dimension von 8 mm Maximum und eine kleinste Dimension von 0,3 mm Minimum aufweist, wobei sein Durchmesser oder seine Breite 1 bis 8 mm, bevorzugt 2 bis 5 mm beträgt, und seine Höhe 0,3 bis 5 mm, bevorzugt 0,5 bis 4 mm beträgt.

13. Verfahren zur Herstellung eines rohen Presskörpers wie in den Ansprüchen 1 bis 12 definiert, umfassend die Schritte:
- Mischen einer bindenden Phase aus einem oder mehreren pulverförmigen Bindemitteln, wobei der rohe Presskörper (3) die Eigenschaft aufweist, nach Sättigung mit einer Hydratisierungsflüssigkeit mit den Bindemitteln zu reagieren, um ein chemisch gebundenes keramisches Material zu bilden, einem oder mehreren Expansions-kompensierenden Additiven, die geeignet sind, um dem Material dimensionsstabile Langzeiteigenschaften zu verleihen, wobei die Additive ausgewählt worden sind aus der Gruppe bestehend aus einem oder mehreren Additiven in der Gruppe, bestehend aus porösen Teilchen oder porösen Aggregaten, weichen Teilchen, die einen E-Modul aufweisen, der niedriger als der E-Modul der bindenden Phase ist, und einer sekundären Phase, wobei die sekundäre Phase mit der bindenden Phase reagiert, wobei die Expansions-kompensierenden Additive in einer Menge von ungefähr 1 bis 20 Volumenprozent des rohen Presskörpers vorhanden sind;
- Formen eines Pulverkörpers aus dem pulverförmigen Material und den Additiven;
- Aussetzen des Pulverkörpers einem Druck von 40 bis 150 MPa und einer Temperatur von Raumtemperatur bis mindestens 150°C derart, dass ohne Sinterreaktionen ein dicht verbundener roher Presskörper (3) mit einem Verdichtungsgrad von 55 bis 67 Volumenprozent der festen Phase erhalten wird.

14. Verfahren gemäß Anspruch 13, **dadurch gekennzeichnet, dass** der rohe Presskörper (3) im Wesentlichen frei von Füllstoffteilchen, die Härte bereitstellen, ist.

15. Verfahren gemäß Anspruch 13, **dadurch gekennzeichnet, dass** der rohe Presskörper (3) bis zu 50 Volumenprozent Ballastmaterial umfasst.

16. Verfahren gemäß einem der Ansprüche 13 bis 15, **dadurch gekennzeichnet, dass** die bindende Phase mindestens im Wesentlichen aus Calciumaluminatzement besteht.

17. Verfahren gemäß einem der Ansprüche 13 bis 16, **dadurch gekennzeichnet, dass** bei dem rohen Presskörper (3) ein Verdichtungsgrad von 57 bis 63 Volumenprozent der festen Phase, vorzugsweise 58 bis 61 Volumenprozent der festen Phase, herbeigeführt wird.

18. Verfahren gemäß einem der Ansprüche 13 bis 17, **dadurch gekennzeichnet, dass** die Additive mindestens im Wesentlichen aus einer sekundären Phase, wobei die sekundäre Phase vorzugsweise aus OPC-Zement und/oder feinem kristallinem Siliciumdioxid und/oder einer anderen Si-haltigen Phase besteht, bevorzugt in einer Gesamtmenge von 1 bis 20 Volumenprozent und noch stärker bevorzugt 1 bis 10 Volumenprozent im rohen Presskörper bestehen.

19. Verfahren gemäß Anspruch 18, **dadurch gekennzeichnet, dass** die sekundäre Phase aus OPC-Zement in einer Menge von 1 bis 5 Volumenprozent und/oder feinem kristallinen Siliciumdioxid in einer Menge von 1 bis 5 Volumenprozent besteht.

20. Verfahren gemäß einem der Ansprüche 13 bis 19, **dadurch gekennzeichnet, dass** die Additive mindestens im Wesentlichen aus porösen Teilchen oder porösen Aggregaten bestehen, welche mindestens im Wesentlichen aus Oxiden von Al, Zr, Ti, Si oder Zn und Sn bestehen, wobei diese vorzugsweise einen Durchmesser von 2 bis 30 µm, noch stärker bevorzugt 5 bis 15 µm, eine offene Porosität von 20 bis 60 %, bevorzugt 30 bis 50 % aufweisen, und wobei die Porenöffnungen in den Teilchen/Aggregaten weniger als 5 µm, bevorzugt 0,1 bis 5 µm und noch stärker bevorzugt 1 bis 3 µm aufweisen.

21. Verfahren gemäß einem der Ansprüche 13 bis 19, **dadurch gekennzeichnet, dass** die Additive mindestens im Wesentlichen aus porösen Teilchen bestehen, wobei die porösen Teilchen mindestens im Wesentlichen aus Mikrokügelchen mit einer hohen geschlossenen Porosität bestehen, wobei die Mikrokügelchen vorzugsweise aus Glas sind und eine Porosität aufweisen, die 50 % übersteigt, vorzugsweise 80 % übersteigt, und in Mengen von weniger als 2 Volumenprozent des rohen Presskörpers vorhanden sind.

22. Verfahren gemäß einem der Ansprüche 13 bis 21, **dadurch gekennzeichnet, dass** der rohe Presskörper (3) einen Verdichtungsgrad von 57 bis 63 Volumenprozent der festen Phase, bevorzugt 58 bis 61 Volumenprozent der festen Phase, aufweist.

23. Verfahren gemäß einem der Ansprüche 13 bis 22, **dadurch gekennzeichnet, dass** der rohe Presskörper (3) außerdem einen Beschleuniger für die beschleunigte Reaktion zwischen den Bindemitteln und der Hydratisierungsflüssigkeit, wobei der Beschleuniger vorzugsweise aus einem Salz eines Alkalimetalls besteht, in einer Menge von 0,1 bis 0,5 Volumenpromille, bevorzugt 0,2 bis 0,3 Volumenpromille, bezogen auf den Feststoffgehalt, umfasst.

24. Verfahren gemäß einem der Ansprüche 13 bis 23, **dadurch gekennzeichnet, dass** die Bindemittel im Wesentlichen eine Korngröße von 2 bis 8 µm, bevorzugt 3 bis 4 µm aufweisen.

25. Verfahren gemäß einem der Ansprüche 13 bis 24, **dadurch gekennzeichnet, dass** der rohe Presskörper (3) so hergestellt wird, dass er eine größte Dimension von 8 mm Maximum und eine kleinste Dimension von 0,3 mm Minimum aufweist, wobei sein Durchmesser oder seine Breite 1 bis 8 mm, bevorzugt 2 bis 5 mm beträgt, und seine Höhe 0,3 bis 5 mm, bevorzugt 0,5 bis 4 mm beträgt.

26. Verfahren gemäß einem der Ansprüche 13 bis 25, **dadurch gekennzeichnet, dass** das Verfahren einen Tablettenpressschritt für jeden rohen Presskörper (3), der hergestellt wird, umfasst, wobei das Tablettenpressen bei einem Druck von 40 bis 150 MPa, vorzugsweise 70 bis 110 MPa, durchgeführt wird.

27. Verfahren gemäß einem der Ansprüche 13 bis 26, **dadurch gekennzeichnet, dass** der rohe Presskörper (3) dadurch geformt wird, indem die Verdichtung in einem Modell (6) eines Hohlraums (5), in den der rohe Presskörper eingesetzt werden soll, ausgeführt wird.

28. Verfahren gemäß einem der Ansprüche 13 bis 27, **dadurch gekennzeichnet, dass** die Pulvermischung und/oder der rohe Presskörper (3) bei Temperaturen oberhalb von 150°C vorkonditioniert wird.

29. Gehärtetes Material auf Basis des in den Ansprüchen 1 bis 12 definierten, rohen Presskörpers in hydratisierter Form.

30. Gehärtetes Material gemäß Anspruch 29, **dadurch gekennzeichnet, dass** das Material eine Druckfestigkeit von 170 bis 200 MPa besitzt.

31. Gehärtetes Material gemäß Anspruch 29 oder 30, **dadurch gekennzeichnet, dass** das Material eine Vickers-Härte von 110 bis 130 besitzt.

32. Verfahren zur Herstellung des in den Ansprüchen 29 bis 31 definierten gehärteten Materials, umfassend den Schritt des in Kontakt bringens eines in den Ansprüchen 1 bis 12 definierten rohen Presskörpers mit einer Hydratisierungsflüssigkeit.

33. Verfahren gemäß Anspruch 32, **dadurch gekennzeichnet, dass** die Hydratisierungsflüssigkeit veranlasst wird, den rohen Presskörper (3) zu durchdringen, indem der rohe Presskörper mindestens teilweise in die Hydratisierungsflüssigkeit eingetaucht wird, vorzugsweise für mindestens 5 bis 15 Sekunden, bevorzugt für mindestens 10 Sekunden und bis zu 30 Sekunden, so dass es der Hydratisierungsflüssigkeit ermöglicht wird, durch die im rohen Presskörper wirkenden Kapillarkräfte absorbiert zu werden, wobei der rohe Presskörper im Zusammenhang mit seinem in die Hydratisierungsflüssigkeit eingetaucht werden vorzugsweise mit Hilfe eines Insertionsinstrumentes (7) gegriffen wird und dann jegliche auf dem rohen Presskörper verbleibende überschüssige Hydratisierungsflüssigkeit abgedampft wird.

34. Verfahren gemäß Anspruch 32 oder 33, **dadurch gekennzeichnet, dass** die Hydratisierungsflüssigkeit vorzugsweise einen Beschleuniger für die Reaktion zwischen der bindenden Phase und der Hydratisierungsflüssigkeit, wobei der Beschleuniger vorzugsweise aus einem Salz eines Alkalimetalls besteht, vorzugsweise in einer Menge von 0,1 bis 1 g/l der Flüssigkeit, bevorzugt mindestens 0,2 g/l, noch stärker bevorzugt mindestens 0,4 g/l und am stärksten bevorzugt mindestens 0,8 g/l der Flüssigkeit, enthält.

35. Verfahren gemäß Anspruch 33, **dadurch gekennzeichnet, dass** ein erster roher Presskörper (3) durch die Hydratisierungsflüssigkeit getränkt wird, um dann in einen Hohlraum (5) gepackt zu werden, vorzugsweise mit Hilfe eines Befüllinstruments, woraufhin ein zweiter roher Presskörper (3) mit der Hydratisierungsflüssigkeit getränkt wird, um dann in den Hohlraum (5) gepackt zu werden, vorzugsweise mit Hilfe des Befüllinstruments, woraufhin weitere rohe Presskörper mit der Hydratisierungsflüssigkeit getränkt werden, um dann in den Hohlraum gepackt zu werden, vorzugsweise mit Hilfe des Befüllinstruments, vorzugsweise bis der Hohlraum mit feuchten, zusammengedrückten rohen Presskörper gefüllt ist, welchen dann gestattet wird, zu dem chemisch gebundenen keramischen Material zu härten.

36. Verfahren gemäß Anspruch 35, **dadurch gekennzeichnet, dass** das abschließende Verfüllen und das Entfernen jeglichen Überschusses an Hydratisierungsflüssigkeit in situ mit Hilfe einer Verdichtungsvorrichtung (7) durchgeführt wird - einem Werkzeug - wobei dessen Bauteil (19), welches auf die feuchten zusammengedrückten, rohen Presskörper einwirkt, aus einem harten porösen Material besteht, in welches jeglicher Überschuss an Hydratisierungsflüssigkeit absorbiert wird, gleichzeitig während die feuchten Presskörper (3), die zusammengepackt werden, im Hohlraum (5) weiter verdichtet werden.

37. Verfahren gemäß Anspruch 35 oder 36, **dadurch gekennzeichnet, dass** nachfolgendes Polieren einer freien Oberfläche des gebildeten, chemisch gebundenen keramischen Materials vorzugsweise innerhalb von 3 bis 10 Minuten, noch stärker bevorzugt innerhalb von 3 bis 7 Minuten, nachdem die Verdichtung der feuchten rohen Presskörper (3) abgeschlossen wurde, ausgeführt wird.

38. Dentales Befüllmaterial, umfassend den rohen Presskörper gemäß einem der Ansprüche 1 bis 12 oder das gehärtete Material gemäß den Ansprüchen 29 bis 31.

39. Elektronische Schaltung, Biosensor und optische Faser mit einem Träger aus einem Material, umfassend den rohen Presskörper gemäß einem der Ansprüche 1 bis 12 oder das gehärtete Material gemäß den Ansprüchen 29 bis 31.

40. Verwendung des rohen Presskörpers gemäß einem der Ansprüche 1 bis 12 oder des gehärteten Materials gemäß den Ansprüchen 29 bis 31 als ein Trägermaterial in einer mikromechanischen Anwendung.

## Revendications

1. Comprimé brut (3) comprenant une phase liante d'un ou plusieurs agents liants en poudre, lequel comprimé brut a la capacité, après saturation avec un liquide d'hydratation, de réagir avec les agents liants pour former un matériau céramique à liaison chimique, lequel comprimé brut (3) comprend également un ou plusieurs additifs à compensation de dilatation adaptés pour donner au matériau des propriétés à long terme dimensionnellement stables, dans lequel lesdits additifs ont été choisis dans le groupe constitué par un ou plusieurs additifs du groupe comprenant les particules poreuses ou les agrégats poreux, les particules malléables ayant un module E inférieur au module E de la phase liante, et une phase secondaire, laquelle phase secondaire réagit avec la phase liante, dans lequel les additifs à compensation de dilatation sont présents dans une quantité d'environ 1 à 20 pour cent en volume du comprimé brut, **caractérisé en ce que** le comprimé brut a un degré de compacité de 55 à 67 pour cent en volume de phase solide.

2. Comprimé brut selon la revendication 1, **caractérisé en ce que** le comprimé brut (3) est sensiblement exempt de particules de remplissage conférant une certaine dureté.

3. Comprimé brut selon la revendication 1, **caractérisé en ce que** le comprimé brut (3) comprend jusqu'à 50 pour cent en volume de matériau de ballast.

4. Comprimé brut selon l'une quelconque des revendications précédentes, **caractérisé en ce que** ladite phase liante est constituée au moins sensiblement de ciment d'aluminate de calcium.

5. Comprimé brut selon l'une quelconque des revendications précédentes, **caractérisé en ce que** lesdits additifs sont constitués au moins sensiblement d'une phase secondaire, ladite phase secondaire étant de préférence constituée de ciment OPC et/ou de silice cristalline fine et/ou d'une autre phase contenant du Si, de préférence dans une quantité totale de 1 à 20 pour cent en volume et encore plus préférablement de 1 à 10 pour cent en volume dans le comprimé brut.

6. Comprimé brut selon la revendication 5, **caractérisé en ce que** ladite phase secondaire est constituée de ciment OPC dans une quantité de 1 à 5 pour cent en volume et/ou de silice cristalline fine dans une quantité de 1 à 5 pour cent en volume.

7. Comprimé brut selon l'une quelconque des revendications 1 à 6, **caractérisé en ce que** lesdits additifs sont constitués au moins sensiblement de particules poreuses ou d'agrégats poreux, lesquels sont constitués au moins sensiblement d'oxydes d'Al, Zr, Ti, Si ou ZN et Sn, ceux-ci ayant de préférence un diamètre de 2 à 30 µm, encore plus préférablement de 5 à 15 µm, une porosité ouverte de 20 à 60 %, de préférence de 30 à 50 %, et les ouvertures des pores dans les particules/agrégats étant inférieures à 5 µm, de préférence de 0,1 à 5 µm et encore plus préférablement de 1 à 3 µm.

8. Comprimé brut selon l'une quelconque des revendications 1 à 6, **caractérisé en ce que** lesdits additifs sont constitués au moins sensiblement de particules poreuses, lesquelles particules poreuses sont constituées au moins sensiblement de microsphères ayant une porosité fermée élevée, lesquelles microsphères sont de préférence en verre et ont une porosité supérieure à 50 %, de préférence supérieure à 80 %, et sont présentes dans des quantités inférieures à 2 pour cent en volume du comprimé brut.

9. Comprimé brut selon l'une quelconque des revendications précédentes, **caractérisé en ce que** ledit comprimé brut (3) a un degré de compacité de 57 à 63 pour cent en volume de phase solide, de préférence de 58 à 61 pour cent en volume de phase solide.

10. Comprimé brut selon l'une quelconque des revendications précédentes, **caractérisé en ce que** ledit comprimé brut (3) comprend également un accélérateur pour la réaction accélérée entre lesdits agents liants et le liquide d'hydratation, ledit accélérateur étant de préférence constitué d'un sel d'un métal alcalin de préférence d'un sel d'un métal alcalin, dans une quantité de 0,1 à 0,5 par mil en volume, de préférence de 0,2 à 0,3 par mil en volume, calculée d'après la teneur en matière solide.

11. Comprimé brut selon l'une quelconque des revendications précédentes, **caractérisé en ce que** lesdits agents liants ont sensiblement une granulométrie de 2 à 8 µm, de préférence de 3 à 4 µm.

12. Comprimé brut selon l'une quelconque des revendications précédentes, **caractérisé en ce que** ledit comprimé brut (3) a une dimension maximale de 8 mm et une dimension minimale de 0,3 mm, son diamètre ou sa largeur étant de 1 à 8 mm, de préférence de 2 à 5 mm, et sa hauteur étant de 0,3 à 5 mm, de préférence de 0,5 à 4 mm.

13. Procédé de production d'un comprimé brut tel que défini dans les revendications 1 à 12, comprenant les étapes consistant à :
- mélanger une phase liante d'un ou plusieurs agents liants en poudre, lequel comprimé brut (3) a la capacité, après saturation avec un liquide d'hydratation, de réagir avec les agents liants pour former un matériau céramique à liaison chimique, un ou plusieurs additifs à compensation de dilatation adaptés pour donner au matériau des propriétés à long terme dimensionnellement stables, dans lequel lesdits additifs ont été choisis dans le groupe constitué par un ou plusieurs additifs du groupe comprenant les particules poreuses ou les agrégats poreux, les particules malléables ayant un module E inférieur au module E de la phase liante, et une phase secondaire, laquelle phase secondaire réagit avec la phase liante, dans lequel les additifs à compensation de dilatation sont présents dans une quantité d'environ 1 à 20 pour cent en volume du comprimé brut ;
- former un corps en poudre constitué dudit matériau en poudre et desdits additifs ;
- exposer le corps en poudre à une pression de 40 à 150 MPa et à une température allant de la température ambiante à au moins 150 °C, de telle sorte qu'un comprimé brut à haute densité (3), ayant un degré de compacité de 55 à 67 pour cent en volume de phase solide, est obtenu sans réactions de frittage.

14. Procédé selon la revendication 13, **caractérisé en ce que** le comprimé brut (3) est sensiblement exempt de particules de remplissage conférant une certaine dureté.

15. Procédé selon la revendication 13, **caractérisé en ce que** le comprimé brut (3) comprend jusqu'à 50 pour cent en volume de matériau de ballast.

16. Procédé selon l'une quelconque des revendications 13 à 15, **caractérisé en ce que** ladite phase liante est constituée au moins sensiblement de ciment d'aluminate de calcium.

17. Procédé selon l'une quelconque des revendications 13 à 16, **caractérisé en ce que** ledit comprimé brut (3) est produit de façon à présenter un degré de compacité de 57 à 63 pour cent en volume de phase solide, de préférence de 58 à 61 pour cent en volume de phase solide.

18. Procédé selon l'une quelconque des revendications 13 à 17, **caractérisé en ce que** lesdits additifs sont constitués au moins sensiblement d'une phase secondaire, ladite phase secondaire étant de préférence constituée de ciment OPC et/ou de silice cristalline fine et/ou d'une autre phase contenant du Si, de préférence dans une quantité totale de 1 à 20 pour cent en volume et encore plus préférablement de 1 à 10 pour cent en volume dans le comprimé brut.

19. Procédé selon la revendication 18, **caractérisé en ce que** ladite phase secondaire est constituée de ciment OPC dans une quantité de 1 à 5 pour cent en volume et/ou de silice cristalline fine dans une quantité de 1 à 5 pour cent en volume.

20. Procédé selon l'une quelconque des revendications 13 à 19, **caractérisé en ce que** lesdits additifs sont constitués au moins sensiblement de particules poreuses ou d'agrégats poreux, lesquels sont constitués au moins sensiblement d'oxydes d'Al, Zr, Ti, Si ou ZN et Sn, ceux-ci ayant de préférence un diamètre de 2 à 30 µm, encore plus préférablement de 5 à 15 µm, une porosité ouverte de 20 à 60 %, de préférence de 30 à 50 %, et les ouvertures des pores dans les particules/agrégats étant inférieures à 5 µm, de préférence de 0,1 à 5 µm et encore plus préférablement de 1 à 3 µm.

21. Procédé selon l'une quelconque des revendications 13 à 19, **caractérisé en ce que** lesdits additifs sont constitués au moins sensiblement de particules poreuses, lesquelles particules poreuses sont constituées au moins sensiblement de microsphères ayant une porosité fermée élevée, lesquelles microsphères sont de préférence en verre et ont une porosité supérieure à 50 %, de préférence supérieure à 80 %, et sont présentes dans des quantités inférieures à 2 pour cent en volume du comprimé brut.

22. Procédé selon l'une quelconque des revendications 13 à 21, **caractérisé en ce que** ledit comprimé brut (3) a un degré de compacité de 57 à 63 pour cent en volume de phase solide, de préférence de 58 à 61 pour cent en volume de phase solide.

23. Procédé selon l'une quelconque des revendications 13 à 22, **caractérisé en ce que** ledit comprimé brut (3) comprend également un accélérateur pour la réaction accélérée entre lesdits agents liants et le liquide d'hydratation, ledit accélérateur étant de préférence constitué d'un sel d'un métal alcalin, dans une quantité de 0,1 à 0,5 par mil en volume, de préférence de 0,2 à 0,3 par mil en volume, calculée d'après la teneur en matière solide.

24. Procédé selon l'une quelconque des revendications 13 à 23, **caractérisé en ce que** lesdits agents liants ont sensiblement une granulométrie de 2 à 8 µm, de préférence de 3 à 4 µm.

25. Procédé selon l'une quelconque des revendications 13 à 24, **caractérisé en ce que** ledit comprimé brut (3) est produit de façon à présenter une dimension maximale de 8 mm et une dimension minimale de 0,3 mm, son diamètre ou sa largeur étant de 1 à 8 mm, de préférence de 2 à 5 mm, et sa hauteur étant de 0,3 à 5 mm, de préférence de 0,5 à 4 mm.

26. Procédé selon l'une quelconque des revendications 13 à 25, **caractérisé en ce que** ledit procédé comprend une étape de compression des comprimés pour chaque comprimé brut (3) produit, la compression des comprimés étant effectuée à une pression de 40 à 150 MPa, de préférence de 70 à 110 MPa.

27. Procédé selon l'une quelconque des revendications 13 à 26, **caractérisé en ce que** ledit comprimé brut (3) est formé de telle sorte que ledit compactage est effectué dans un modèle (6) d'une cavité (5) dans laquelle le comprimé brut doit être introduit.

28. Procédé selon l'une quelconque des revendications 13 à 27, **caractérisé en ce que** ledit mélange en poudre et/ou ledit comprimé brut (3) est préconditionné à des températures supérieures à 150 °C.

29. Matériau durci à base du comprimé brut défini dans les revendications 1 à 12, sous forme hydratée.

30. Matériau durci selon la revendication 29, **caractérisé en ce que** ledit matériau a une résistance à la compression de 170 à 200 MPa.

31. Matériau durci selon la revendication 29 ou 30, **caractérisé en ce que** ledit matériau a une dureté Vickers de 110 à 130.

32. Procédé de production du matériau durci défini dans les revendications 29 à 31, comprenant l'étape consistant à mettre en contact un comprimé brut défini dans les revendications 1 à 12 avec un liquide d'hydratation.

33. Procédé selon la revendication 32, **caractérisé en ce que** ledit liquide d'hydratation sature le comprimé brut (3) de telle sorte que le comprimé brut est immergé au moins partiellement dans le liquide d'hydratation, de préférence pendant au moins 5 à 15 secondes, de préférence au moins 10 secondes et jusqu'à 30 secondes, de telle manière que le liquide d'hydratation est absorbé par les forces capillaires agissant dans le comprimé brut, le comprimé brut étant de préférence saisi par un instrument d'insertion (7) en connexion avec celui-ci étant immergé dans le liquide d'hydratation et tout liquide d'hydratation superficiel restant sur le comprimé brut étant ensuite séché.

34. Procédé selon la revendication 32 ou 33, **caractérisé en ce que** ledit liquide d'hydratation contient de préférence un accélérateur pour la réaction entre la phase liante et le liquide d'hydratation, ledit accélérateur étant de préférence constitué d'un sel d'un métal alcalin, de préférence dans une quantité de 0,1 à 1 g/l de liquide, de préférence d'au moins 0,2 g/l, encore plus préférablement d'au moins 0,4 g/l et le plus préférablement d'au moins 0,8 g/l de liquide.

35. Procédé selon la revendication 33, **caractérisé en ce qu'**un premier comprimé brut (3) est saturé par le liquide d'hydratation, pour être ensuite introduit dans une cavité (5), de préférence à l'aide d'un outil de remplissage, après quoi un deuxième comprimé brut (3) est saturé par le liquide d'hydratation, pour être ensuite introduit dans ladite cavité (5), de préférence à l'aide dudit outil de remplissage, après quoi tout autre comprimé brut est saturé par le liquide d'hydratation, pour être ensuite introduit dans ladite cavité (5), de préférence à l'aide dudit outil de remplissage, de préférence jusqu'à ce que la cavité soit remplie de comprimés bruts compressés et humides, qui sont ensuite durcis en ledit matériau céramique à liaison chimique.

36. Procédé selon la revendication 35, **caractérisé en ce que** le garnissage final et le retrait de tout excédent de liquide d'hydratation sont effectués in situ à l'aide d'un dispositif de compactage (7) - un outil - dont la partie (19) agissant contre les comprimés bruts compressés humides est constitué d'un matériau poreux dur dans lequel tout excédent de liquide d'hydratation est absorbé, alors que les comprimés bruts humides (3) introduits ensemble sont compressés dans la cavité (5) encore plus.

37. Procédé selon la revendication 35 ou 36, **caractérisé en ce que** le polissage ultérieur d'une surface libre du matériau céramique à liaison chimique formé est effectué, de préférence dans les 3 à 10 minutes, encore plus préférablement dans les 3 à 7 minutes suivant le compactage des comprimés bruts humides (3).

38. Matériau d'obturation dentaire comprenant le comprimé brut selon l'une quelconque des revendications 1 à 12 ou le matériau durci selon les revendications 29 à 31.

39. Circuit électronique, biocapteur et fibre optique ayant un support constitué d'un matériau comprenant le comprimé brut selon l'une quelconque des revendications 1 à 12 ou le matériau durci selon les revendications 29 à 31.

40. Utilisation du comprimé brut selon l'une quelconque des revendications 1 à 12 ou le matériau durci selon les revendications 29 à 31 en tant que matériau support dans une application micromécanique.
